# EUROPEAN PATENT APPLICATION

(11) **EP 1 468 642 A1**
(43) Date of publication of application: **20.10.2004**
(21) Application number: 04007072.4
(22) Date of filing: 24.03.2004
(51) Int. Cl.: A61B 5/022

(54) **Blood pressure meter cuff fastener and electronic blood pressure meter**

(30) Priority: 18.04.2003 JP 2003113698
(71) Applicant: Omron Healthcare Co., Ltd., Kyoto 615-0084 (JP)
(72) Inventor: Yoshihico, Sano, Ukyo-ku Kyoto-shi Kyoto 615-00084 (JP); Osamu, Shirasaki, Ukyo-ku Kyoto-shi Kyoto 615-00084 (JP); Tomonori, Inoue, Ukyo-ku Kyoto-shi Kyoto 615-00084 (JP); Toshio, Ohtani, Ukyo-ku Kyoto-shi Kyoto 615-00084 (JP)
(74) Representative: Kilian, Helmut, Dr.

(57) **Abstract**

Provided is a blood pressure meter cuff fastener having a function capable of switching between a winding length of a blood pressure meter cuff fastener in measurement of a blood pressure and a winding length of the blood pressure meter cuff fastener which is mounted for a long time other than measurement of a blood pressure with ease. A blood pressure meter cuff fastener has a first body section 102, a second body section 103 provided pivotably around a pivot section 142 on the other end side of the first body section 102 so as to be folded on the first body section 102 , and a third body section 104 provided pivotably around a pivot section 141 on the other side of the second body section 103 from the side on which the first body section 102 thereof is provided so as to be folded on the second body section 103, and selection can be easily conducted of one of three states including a measuring winding length state, a non-measuring winding state and a mount/demount length state.

## Description

### BACKGROUND OF THE INVENTION

### 1. Field of the Invention

The present invention relates to a blood pressure meter used in a blood pressure meter measuring a blood pressure while being mounted on a human body, and in particular, to a blood pressure meter cuff fastener used in fastening a blood pressure meter cuff on a human body and an electronic blood pressure meter using the same.

### 2. Description of the Prior Art

In order to sustain a blood pressure measuring precision, it is required for a cuff with a pressurizing fluid bag used in pressurizing a blood vessel to be close contact with a measuring site and for measurement to be made at a predetermined winding force at all times. Therefore, it is required for a winding length (winding force) of a belt member, if once determined, to be enabled to be fixed, thereby reproducing the winding length of the belt suited for the person. Bloodpressure meter cuff fasteners with a mechanism for reproducing the winding length of the belt have been disclosed, for example, in the following documents including Patent literature 1, Patent literature 2 and Patent literature 3, which correspond to JP-A Nos. 11-318, 10-33490 and 10-127588, respectively.

In the conventional blood pressure meter cuff fasteners described above, consideration has been given to reproducibility of a winding length of a belt member only in measurement. In a blood pressure meter cuff fastener for a carrying blood pressure meter capable of being mounted for a long duration, however, a necessity arises for keeping a cuff to be mounted on a human body for a long time even while no measurement is made. In this context, if a winding length at which a cuff is in close contact with a human body during measurement is kept for a long time other than measurement of a blood pressure, a burden is imposed on a user that pressurization with a winding force to a measuring site of the user is kept long.

Hence, it is required to conduct fine adjustment such as relaxation of a winding length (winding force) or the like while no measurement is made. When measurement restarts, it is required to adjust the relaxed winding length to an original winding length in measurement, whereas a problem occurs that it is impossible to reproduce the original winding length with correctness.

### SUMMARY OF THE INVENTION

The invention has been made to solve the problem and it is an object of the invention to provide a blood pressure meter cuff fastener having a function capable of switching between a winding length of a blood pressure meter cuff fastener in measurement of a blood pressure and a winding length of the blood pressure meter cuff fastener in non-measurement of a blood pressure with ease and an electronic blood pressure meter using the same.

An aspect of a blood pressure meter cuff fastener based on the invention is directed to a blood pressure meter cuff fastener used for fastening a blood pressure meter cuff on a human body constructed in the following way.

Abloodpressuremetercuff fastener based on the invention includes: a belt member wound around a part of a human body and winding length adjusting unit, connected to the belt member, and for adjusting a winding length of the blood pressure meter cuff fastener with the belt member thereof around the part of a human body, wherein the winding length adjusting unit is provided so as to be capable of selecting one of three states including a measuring winding length state adjusting the blood pressure meter cuff fastener to a first winding length for measuring a blood pressure in the part of a human body, a non-measuring winding length state adjusting the blood pressure meter cuff fastener to a second winding length longer than the first winding length in order to maintain a mounting state thereof on the part of a human body in a non-measuring state thereof for a blood pressure and a mount/demount length state capable of mounting or demounting the blood pressure meter cuff fastener on the part of a human body.

With the construction adopted, an easy switching can be effected between the non-measuring winding length state where the blood pressure meter is normally mounted and the measuring winding length state where measurement is made by the blood pressure meter. If the blood pressure meter cuff fastener has been once adjusted to a measuring winding length, the measuring winding length can be, thereafter, reproduced only by switching from the non-measuring winding length state to the measuring winding length state and no necessity arises for conducting fine adjustment of a winding length in each measurement. Since the measuring winding length state can be easily reproduced, reproducibility of a winding length (winding force) in each measurement of a blood pressure can be sustained, thereby enabling improvement on blood pressure measuring precision to be expected.

Since, the non-measuring winding length state can be easily selected, a burden imposed on a human body is alleviated, thereby enabling release from a feeling of restraint of being clamped for a long time.

As a preferred embodiment of the invention, the winding length adjusting unit has the body section and a sliding section slidably provided to the body section, wherein the sliding section is slid in a direction in which the sliding section is accommodated into the body section to thereby enable the measuring winding length state to be acquired, while being slid in a direction in which the sliding section is released from the body section to thereby enable the non-measuring winding length state to be acquired.

As a more preferred embodiment of the invention, the winding length adjusting unit has a first fixing mechanism for selectively fixing one of the measuring winding length state and the non-measuring winding length state between the body section and the sliding section.

As a preferred embodiment of the invention, the body section has a first body section, a second body section provided pivotably on the other end side of the first body section so as to be folded on the first body section, and a third body section provided pivotably on the other side of the second body section from the side on which the first body section thereof is provided so as to be folded on the second body section, wherein the first body section, the second body section and the third body section are folded so as to be superimposed one on another to thereby enable the measuring winding length state and the non-measuring winding length state to be acquired, while being released from the folding state of the first body section, the second body section and the third body section to thereby enable the mount/demount length state to be acquired. As a more preferred embodiment of the invention, a second fixing mechanism for fixing the measuring winding length state and the non-measuring winding length state is providedbetween the first body section and the third body section.

An aspect of the electronic blood pressure meter based on the invention is directed to an electronic blood pressure meter having a blood pressure meter cuff fastener based on any of the above inventions.

### BRIEF DESCRIPTION OF THE DRAWINGS

Fig. 1 shows aperspectiveview showing ameasuringwinding length state of all of a wrist-type blood pressure meter cuff fastener in a first embodiment.
Fig. 2 shows a perspective view showing a non-measuring winding length state of all of the wrist-type blood pressure meter cuff fastener in the first embodiment.
Fig. 3 shows a perspective view showing a mount/demount length state of all of the wrist-type blood pressure meter cuff fastener in the first embodiment.
Fig. 4 shows a partially enlarged view showing an internal structure of a first fixing mechanism in the first embodiment.
Fig. 5 shows a partially enlarged view showing an internal structure of a second fixing mechanism in the first embodiment.
Fig. 6 shows a longitudinal sectional view of a winding length adjusting unit 100B showing a measuring winding length state of a wrist-type blood pressure meter cuff fastener in a second embodiment.
Fig. 7 shows a longitudinal sectional view of the winding length adjusting unit 100B showing a non-measuring winding length state of thewrist-typebloodpressuremetercuff fastener in the second embodiment.
Fig. 8 shows a longitudinal sectional view of the winding length adjusting unit 100B showing a mount/demount length state of the wrist-type blood pressure meter cuff fastener in the second embodiment.
Fig. 9 shows a longitudinal sectional view of a winding length adjusting unit 100C showing a measuring winding length state of a wrist-type blood pressure meter cuff fastener in a third embodiment.
Fig. 10 shows a longitudinal sectional view of the winding length adjusting unit 100C showing a non-measuring winding length state of the wrist-type blood pressure meter cuff fastener in the third embodiment.
Fig. 11 shows a longitudinal sectional view of the winding length adjusting unit 100C showing a mount/demount length state of the wrist-type blood pressure meter cuff fastener in the third embodiment.
Fig. 12 shows a first partially enlarged view showing an internal structure of a first fixing mechanism in the third embodiment.
Fig. 13 shows a second partially enlarged view showing the internal structure of a first fixing mechanism in the third embodiment.
Fig. 14 shows a perspective view showing a measuring winding length state of all of a wrist-type blood pressure meter cuff fastener in a fourth embodiment.
Fig. 15 shows a longitudinal sectional view showing the measuring winding length state of a wrist-type blood pressure cuff fastener in the fourth embodiment.
Fig. 16 shows a perspective view showing a non-measuring winding length state of all of the wrist-type blood pressure meter cuff fastener in the fourth embodiment.
Fig. 17 shows a longitudinal sectional view showing the non-measuring winding length state of the wrist-type blood pressure meter cuff fastener in the fourth embodiment.
Fig. 18 shows a perspective view showing adjustment of a non-measuring winding length of all of the wrist-type blood pressure meter cuff fastener in the fourth embodiment.
Fig. 19 shows a longitudinal sectional view showing adjustment of a non-measuring winding length of the wrist-type blood pressure meter cuff fastener in the fourth embodiment.
Fig. 20 shows a perspective view showing a mount/demount length state of all of the wrist-type blood pressure meter cuff fastener in the fourth embodiment.
Fig. 21 shows a longitudinal sectional view showing the mount/demount length state of the wrist-type blood pressure meter cuff fastener in the fourth embodiment.

### DESCRIPTION OF THE PREFERRED EMBODIMENTS

Description will be given of embodiments of a blood pressure meter cuff fastener based on the invention below with reference to the accompanying drawings. Note that while blood pressure meter cuff fasteners in the following embodiments are blood pressure meter cuff fasteners used in a wrist-type blood pressure meter, any thereof is only an example to which the invention is applied and the invention can be applied widely to other winding-type blood pressure meters.

### (First Embodiment)

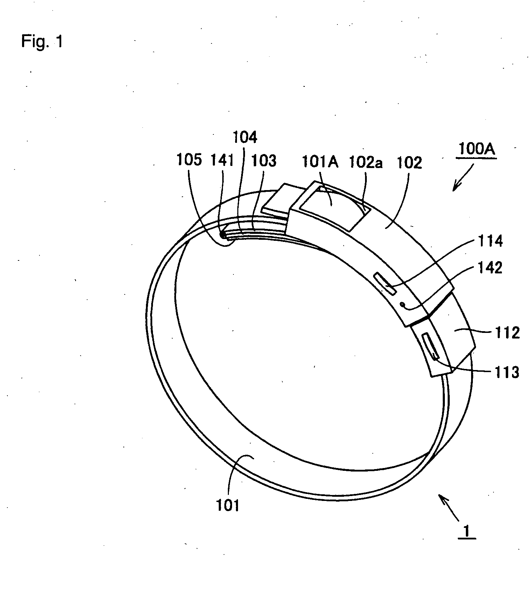

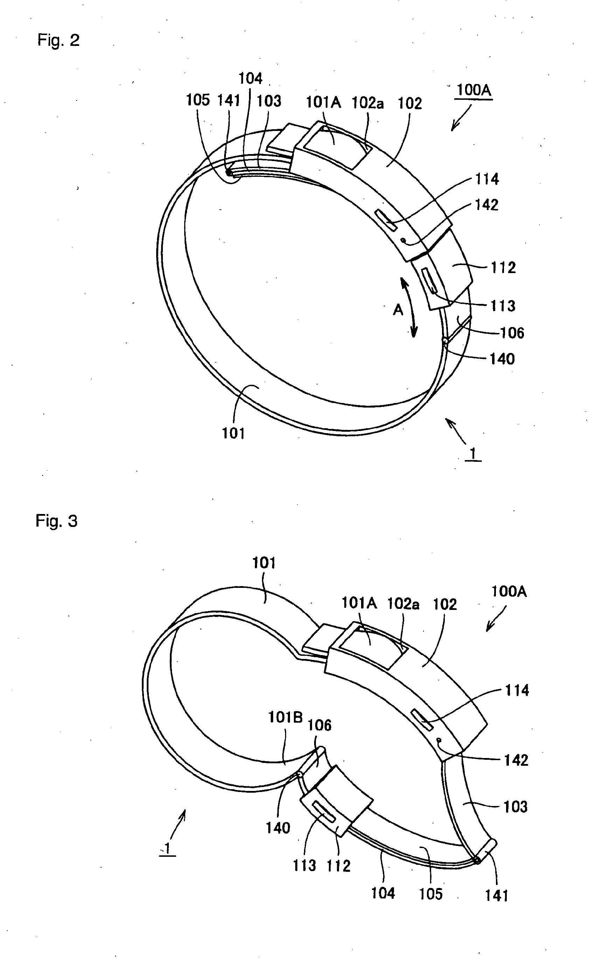

Description will be given of a wrist-type blood pressure meter cuff fastener 1 in the first embodiment with reference to Fig. 1 to Fig. 3. Note that Fig. 1 is a perspective view showing a measuring winding length state of all of a wrist-type blood pressure meter cuff fastener 1 in this embodiment, Fig. 2 is a perspective view showing a non-measuring winding length state of all of the wrist- type blood pressure meter cuff fastener 1 in this embodiment and Fig. 3 is a perspective view showing a mount/demount length state of all of the wrist-type blood pressure meter cuff fastener 1 in this embodiment. Note that the body apparatus of the wrist-type blood pressure meter is not shown in the figure.

### (Structure of Wrist-type Blood Pressure Meter Cuff Fastener 1)

Referring to Fig. 1 to Fig. 3, the wrist-type blood pressure meter cuff fastener 1 used in this embodiment includes: a belt member 101 wound around a wrist of a human body; and a winding length adjusting unit 100A for adjusting a winding length of the blood pressure meter cuff fastener 1 with the belt member 101 thereof around the wrist. The belt member 101 is made of a band-like near ring member.

### [Winding Length Adjusting Unit 100A]

The winding length adjusting unit 100A, as shown in Fig. 3, includes: a first body section 102 having a curved shape; a second body section 103 provided pivotably around a pivot section 142 on the other end side of the first body section 102 so as to be folded on the first body section 102, and a third body section 104 provided pivotably around a pivot section 141 on the other side of the second body section 103 from the side on which the first body section 102 thereof is provided so as to be folded on the second body section 103.

A cuff 105 having a pressurizing fluid bag used for pressurizing a blood vessel in a wrist is attached to the inner side of the third body section 104 (a side in contact with a wrist).

An opening 102a is provided in one side of the first body section 102 and one end side 101A of the belt member 101 is caused to pass through the opening 102a, thereby enabling not only the one end side 101A of the belt member 101 to be fixed, but also length adjustment for the belt member 101 to be realized.

The first body section 102 is providedwith a second fixing mechanism for selecting one of a folded state of the first body section 102, the second body section 103 and the third body section 104, combined, (the state shown in Fig. 1 and Fig. 2) and a state thereof released from the folded state (the state shown in Fig. 3). The detailed structure will be described later.

A sliding section 106 provided slidably on the third body section 104 is attached to the other side of the third body section 104 from the second body section 103. The other side 101B of the belt member 101 provided pivotably around a pivot section 140 is attached to one end of the sliding section 106. Provided to the sliding section 106 is a first fixing mechanism for selectively fixing one of two states including the measuring winding length state adjusting the blood pressure meter cuff fastener 1 to a first winding length for measuring a blood pressure on a wrist (the state shown in Fig. 1) and a non-measuring winding length state adjusting the blood pressure meter cuff fastener 1 to a second winding length longer than the first winding length (the state shown in Fig. 2) in order to exhibit a winding force weaker than in a measuring state, during a non-measurement state for a blood pressure, and to maintain a mounting state on a wrist.

### (Structure of First Fixing Structure)

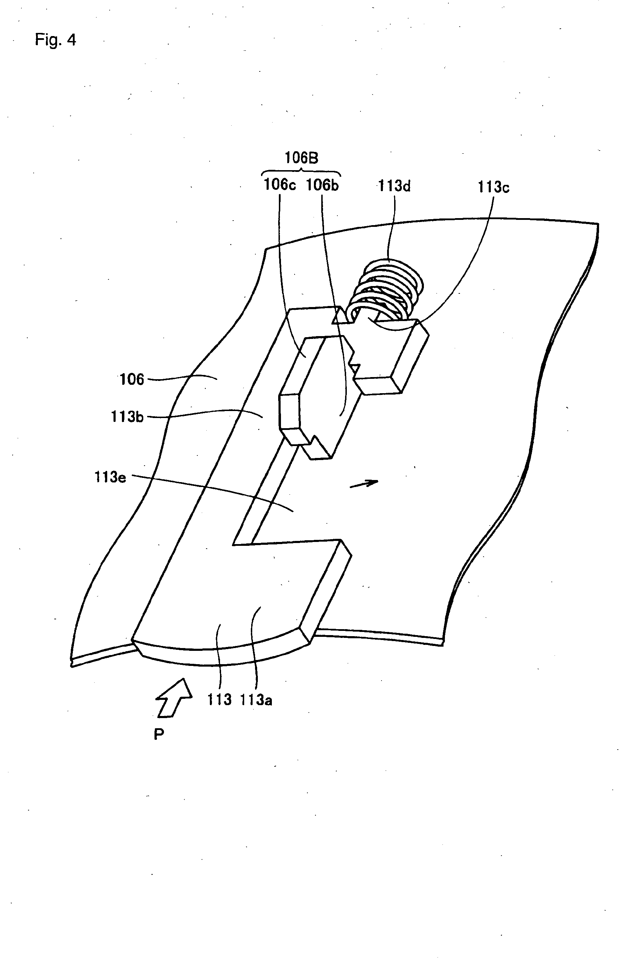

Then, description will be given of the structure of the first fixing mechanism with reference to Fig. 3 and Fig. 4. Note that Fig. 4 is a partially enlarged view showing an internal structure of the first fixing mechanism.

A holding case 112 for slidablyholding the sliding section 106 is attached to the other side of the third body section 104. The holding case 112, as shown in Fig. 4, accommodates a lock cancel button 113 and a coil spring 113d imposes a force on the lock cancel button 113. The sliding section 106 is provided with a lock pin 106B engaging with the lock cancel button 113. The lock pin 106B has a neck section 106b and a head section 106c with the top thereof extending sideways on both sides.

The head section 113a of the lock cancel button 113 is provided so as to protrude outwardly from a sidewall of the holding case 112 and a coil spring 113d is held by a protruding section 113c provided on the other side of the lock cancel button 113a. Note that the other side of the coil spring 113d is provided so as to be brought into contact with the inner surface of a sidewall of the holding case 112.

An engage opening region 113e is provided in a trunk region 113b of the lock cancel button 113, the neck section 106b of the lock pin 106B gets into the engage opening region 113e and the head section 106c of the lock pin 106B engages on the top side of the trunk region 113b.

In the first fixing mechanism with the above construction, since the lock cancel button 113 is, in a normal state, pushed outwardly by a force of the coil spring 113d, the lock pin 106B is, as shown in Fig.4, sustained in a state of engaging in the engage opening region 113e of the lock cancel button 113 to prohibit displacement of the sliding section 106 (the measuring winding length state shown in Fig. 1). On the other hand, in a case where the lock cancel button 113 is pushed inwardly (in a direction of an arrow mark P) , the lock pin 106B is released from engagement with the lock cancel button 113 in the engage opening region 113e, thereby enabling the sliding section 106 to be moved (the non-measuring winding length state shown in Fig. 2)

### (Structure of Second Fixing Structure)

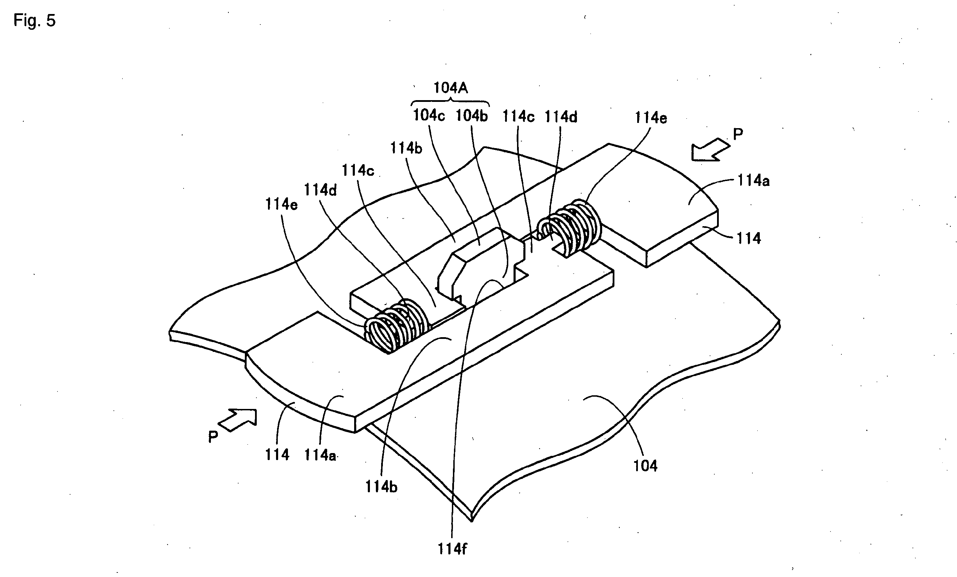

Then, description will be given of the structure of the second fixing mechanism with reference to Fig. 3 and Fig. 5. Note that Fig. 5 is a partially enlarged view showing an internal structure of the second fixing mechanism.

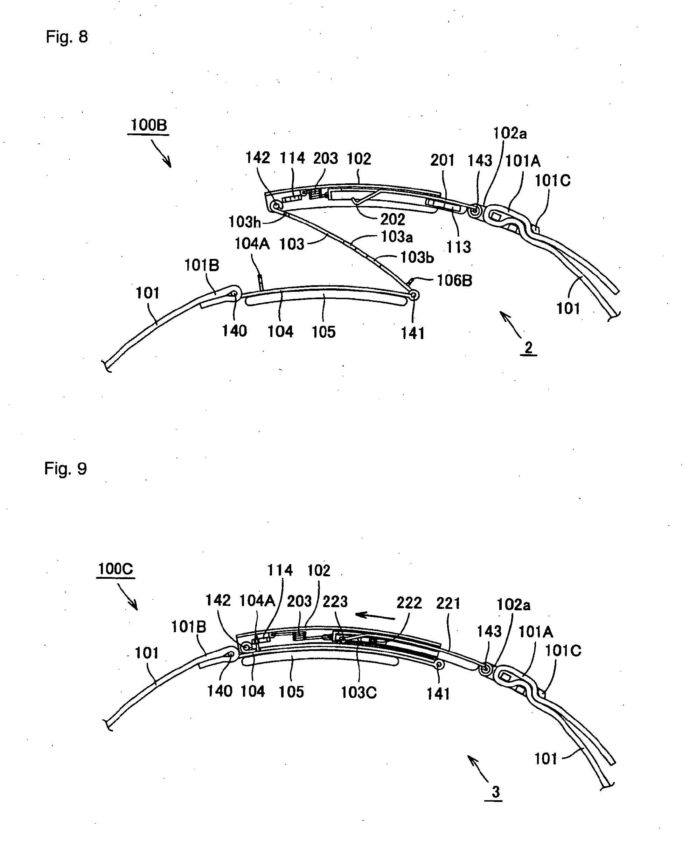

The first body section 102, as shown in Fig. 5, includes: a pair of the lock cancel buttons 114 and 114; and coil springs 114e and 114e imposing forces on the lock cancel buttons 114 and 114. The third body section 104 is provided with a lock pin 104A engaging with the lock cancel buttons 114 and 114. While the lock pin 104A is provided so as to pass through an opening 103h (see Fig. 8 and Fig. 9 described later) formed in the second body section 103 in a state where the first body section 102, the second body section 103 and the third body section 104 are folded one on another, the second body section 103 and the opening 103h are omitted in the figure for the sake of convenience in description.

The lock pin 104A has a neck section 104b; and a head section 104c on the top end thereof extending on both sides.

The head sections 114a and 140a of the lock cancel buttons 114 and 114 are provided so as to protrude outwardly from sidewalls of the first body section 102, and coil springs 114e and 114e are held by protruding sections 114d and 114d provided in leg regions 114c and 114c of the lock cancel buttons 114 and 114. The other ends of the coil springs 114d and 114d are provided so as to be brought into contact with the inner surfaces of the head sections 114a and 114a of the lock cancel buttons 114 and 114.

An engage opening region 114f defined by a pair of the lock cancel buttons 114 and 114 is provided to the trunk regions 114b and 114b of the lock cancel buttons 114 and 114, the neck section 104b in the lock pin 104A get into the engage opening region 114f and the head section 104c of the lockpin 104A engages on the top sides of the leg regions 114c and 114c.

In the second fixingmechanismwith the above construction, since the lock cancel buttons 114 and 114 both are, in a normal state, pushed outwardly by forces of the coil springs 114e and 114e, a state where the lock pin 104A engages in the engage opening region 114f of the lock cancel buttons 114 and 114 is sustained as in the state shown in Fig. 5 to thereby sustain a state where the first body section 102, the second body section 103 and the third body section 104 are folded one on another (the measuring winding length state and the non-measuring winding length state shown in Fig. 1 and Fig. 2). On the other hand, in a case where the lock cancel buttons 114 and 114 both are pushed inwardly (in a direction of an arrow mark P), the lock pin 104A is released from engagement of the engage opening region 114f with the lock cancel buttons 114 and 114, whereby the first body section 102, the second body section 103 and the third body section 104 are released from the folded state (the mount/demount length state shown in Fig. 3).

### [Operation Procedure]

Description will be given of the operation procedure in a case where the wrist-type blood pressure cuff fastener 1 with the above structure with reference to Fig. 1 to Fig. 3.

### <Measuring Winding Length State>

First of all the lock cancel buttons 114 and 114 of the second fixing mechanism are pushed inwardly to obtain the mount/demount length state of the wrist-type blood pressure meter cuff fastener 1 shown in Fig. 3 to cause a wrist to get into the wrist-type blood pressure meter cuff fastener 1.

The cuff 105 is positioned at a measuring position and not only is a length of the belt member 101 adjusted in the opening 102a, but the first body section 102, the second body section 103 and the third body section 104 are also caused to assume a folded state. In this context, the first body section 102, the second body section 103 and the third body section 104 are fixed into the folded state by the second fixing mechanism. In this step, a length of the belt member 101 is adjusted so that a state is obtained where the siding section 106 is accommodated in the holding case 112. With such an adjustment of the length, the blood pressure meter cuff fastener 1 assumes the measuring winding length state shown in Fig. 1 (the first winding length). Thereafter, a blood pressure is measured with the body apparatus of the wrist-type blood pressure meter.

### <Non-measuring Winding Length State>

Then, the lock cancel button 113 of the first fixing mechanism is operated to cancel a locked state of the sliding section 106 to slide the slide section 106 in a direction in which the sliding section is, as shown in Fig. 2, released from the holding case 112 provided on the third body section 104. By doing so, a winding length becomes longer than the first winding length to cause the blood pressure meter cuff fastener 1 to assume the non-measuring winding length state as shown in Fig. 2 (the second winding length). As a result, a feeling of restraint in a wrist can be eliminated while the wrist-type blood pressure meter cuff fastener 1 is mounted on the wrist.

Note that in a case where a blood pressure is measured for the second time, the sliding section 106 is slid in a direction in which the sliding section 106 is accommodated into the holding case 112 provided in the third body section 104, thereby enabling the first winding length in the measuring winding length state set at the start of measurement to be reproduced with ease.

### <Mount/Demount Length State>

In a case where the wrist-type blood pressure meter cuff fastener 1 is taken way from a wrist, the lock cancel button 114 of the second fixing mechanism is operated to, as shown in Fig. 3, release the first body section 102, the second body section 103 and the third body section 104 from the folded state with the second fixing mechanism. With the release, the wrist-type blood pressure meter cuff fastener 1 can be easily taken away from a wrist.

Note that in a case where the wrist-type blood pressure meter cuff fastener 1 is again mounted on a wrist, the first body section 102, the second body section 103 and the third body section 104 are caused to be into the folded state, thereby enabling the wrist-type blood pressure meter cuff fastener 1 to be mounted on the wrist with ease. In this case, since adjustment of a length of the belt member 101, that is adjustment of a length of the wrist-type blood pressure meter cuff fastener 1 has been conducted already; therefore, it is easy to reproduce the measuring winding length state and the non-measuring winding length state can be reproduced with ease.

### [Action and Effect]

In a wrist-type blood pressure meter cuff fastener 1 in this embodiment, the non-measuring winding length state where the blood pressure meter is normally mounted and the measuring winding length state where measurement is conducted by the blood pressure meter can be switched from each to the other with ease using the first fixing mechanism. If a measuring winding length of a wrist-type blood pressure meter fastener 1 is once fixed, no necessity arises for adjustment of a measuring winding length thereafter. Since the measuring winding length state can be easily reproducedwith the first fixing mechanism, it is possible to sustain reproducibility of a winding length (winding force) in each blood pressure measurement, thereby enabling improvement on measuring precision for blood pressure to realized.

Since the non-measuring winding length state can be selected with ease, a burden imposed on a human body is alleviated, thereby enabling release from a feeling of restraint of being clamped for a long time to be attained. The mount/demount length state of the blood pressure meter can be easily switched to or from a winding length state thereof (in measurement or non-measurement) other than the mount/demount length state with the second fixing mechanism, thereby enabling mounting or demounting of the blood pressure to be ensured with ease.

### (Second Embodiment)

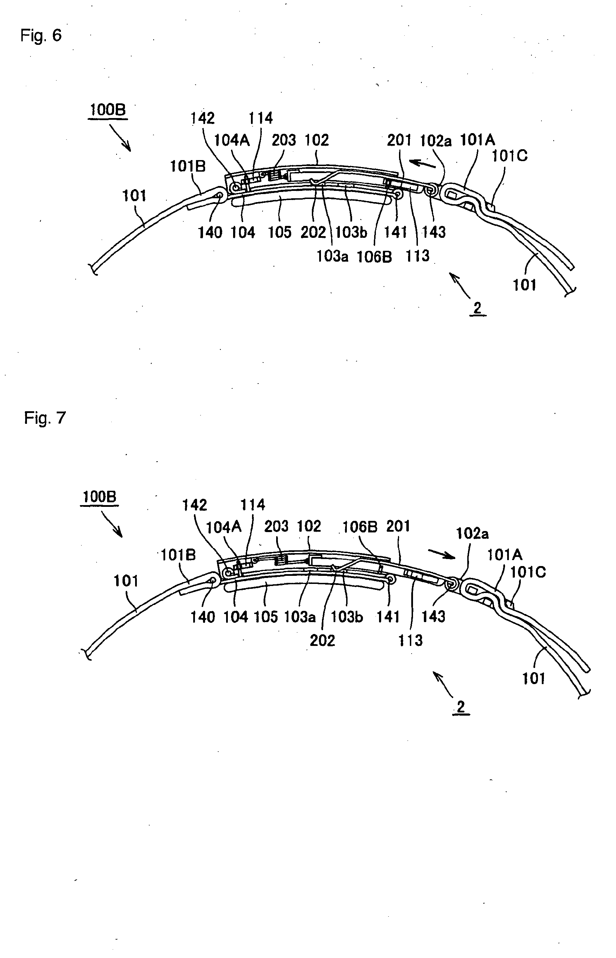

Then, description will be given of a wrist-type blood pressure meter cuff fastener 2 in the second embodiment with reference to Fig. 6 to Fig. 8. The wrist-type blood pressure meter cuff fastener 2 in this embodiment includes: a belt member 101; a winding length ad jus ting unit 100B for adjusting a winding length of the wrist-type blood pressure meter cuff fastener 2 with the belt member 101 around a wrist, and is different from the wrist-type blood pressure meter cuff fastener 1 in the first embodiment in the structure of the winding length adjusting unit 100B. Therefore, detailed description will be given of the structure of the winding length adjusting unit 100B. The same reference marks are attached to the same constitutes as in the winding length adjusting unit 100A and none of descriptions thereof is duplicated.

Note that Fig. 6 is a longitudinal sectional view of the winding length adjusting unit 100B showing a measuring winding lengthstateof thewrist-typebloodpressuremetercuff fastener 2 in this embodiment, Fig. 7 is a longitudinal sectional view of thewinding length ad jus ting unit 100B showing anon-measuring winding length state of the wrist-type blood pressure meter cuff fastener 2 in this embodiment and Fig. 8 is a longitudinal sectional view of the winding length adjusting unit 100B showing a mount/demount length state of the wrist-type blood pressure meter cuff fastener 2 in this embodiment. Note that the body apparatus of the wrist-type blood pressure meter is omitted in the figures.

### [Winding Length Adjusting Unit 100B]

The winding length adjusting unit 100B in this embodiment, as shown in Fig. 8, includes: a first body section 102 having a curved shape; a second body section 103 provided pivotably around a pivot section 142 on the other end side of the first body section 102 so as to be folded on the first body section 102, and a third body section 104 provided pivotably around a pivot section 141 on the other side of the second body section 103 from the side on which the first body section 102 thereof is provided so as to be folded on the second body section 103.

A cuff 105 having a pressurizing fluid bag used for pressurizing a blood vessel in a wrist is attached to the inner side of the third body section 104 (a side in contact with a wrist).

A sliding section 201 slidable relative to the first body section 102 is accommodated in one end side of the first body section 102. A coil spring 203 is connected between one end side of the sliding section 201 and the first body section 102. A connecting member 101C having an opening 102a is connected pivotably around a pivot section 143 to the other side of the sliding section 201. By causing one end side 101A of the belt member 101 to pass through the opening 102a, not only is the one end side 101A of the belt member 101 fixed, but a length of the belt member 101 can also be adjusted.

The sliding section 201 is provided with a lock cancel button 113 of the first fixing mechanism and with an engage tongue-shapedpiece 202 for elastically engaging in engage holes 103a and 103b provided in the second body section 103 in order to fix a sliding position of the sliding section 201.

Lock cancel buttons 114 and 114 of the second fixing mechanism are provided in a region between the pivot section 142 of the first body section 102 and a coil spring 203. Not only the engage holes 103a and 103b are provided in the second body section 103, but also the lock pin 106B of the first fixing mechanism is provided in the vicinity of a pivot section 141 and an opening 103h included as a constituent in the second fixing mechanism is provided in the vicinity of the pivot section 142. The lock pin 104A included as a constituent in the second fixing mechanism is provided in the third body section 104 in the vicinity of a pivot section 140.

Note that detailed structures of the first and second fixing mechanisms are similar to that in the first embodiment; therefore none of descriptions thereof is duplicated.

### [Operation Procedure]

Description will be given of an operation procedure in a case where the wrist-type blood pressure meter cuff fastener 2 with the above structure with reference to Fig. 6 to Fig. 8.

### <Measuring Winding Length State>

First of all, the lock cancel buttons 114 and 114 of the second fixing mechanism are pushed inwardly to obtain the mount/demount length state of the wrist-type blood pressure meter cuff fastener 2 shown in Fig. 8 to cause a wrist to get thereinto. In this step, the sliding section 201 assumes a state of being accommodated in the first body section 102 by a force of the coil spring 203.

The cuff 105 is positioned at a measuring position and not only is a length of the belt member 101 adjusted in the opening 102a, but the first body section 102, the second body section 103 and the third body section 104 are also caused into a folded state. In this context, the first body section 102, the second body section 103 and the third body section 104 are fixed into the folded state by the second fixing mechanism. In this step, the engage tongue-shaped piece 202 is engaged in the engage hole 103a provided in the second body section 103. Thereby, the blood pressure meter cuff fastener 2 assumes the measuring winding length state shown in Fig. 6 (the first winding length). Thereafter, a blood pressure is measured with the body apparatus of the wrist-type blood pressure meter.

### <Non-measuring Winding Length State>

Then, the lock cancel button 113 is operated in the first fixing mechanism to cancel a locked state of the sliding section 201 to slide the slide section 201 in a direction in which the sliding section 201 is, as shown in Fig. 7, released from the first body section 102. In this step, the engage tongue-shaped piece 202 engages in the engage hole 103b provided in the second body section 103. By doing so, a winding length becomes longer than the first winding length to cause the blood pressure meter cuff fastener 2 to assume the non-measuring winding length state shown in Fig. 7 (the second winding length). As a result, a feeling of restraint in a wrist can be eliminated while the wrist-type blood pressure meter cuff fastener 2 is mounted on the wrist.

Note that in a case where a blood pressure is measured for the second time, the sliding section 201 is slid in a direction in which the sliding section 201 is accommodated into the first body section 102, thereby enabling the first winding length in the measuring winding length state set at the start of measurement to be reproduced with ease.

### <Mount/Demount Length State>

In a case where the wrist-type blood pressure meter cuff fastener 2 is taken way from a wrist, the lock cancel button 114 of the second fixing mechanism is operated to, as shown in Fig. 8, release the first body section 102, the second body section 103 and the third body section 104 from the folded state. With the release, the wrist-type blood pressure meter cuff fastener 2 can be easily taken away from a wrist. In this step, since engagement of the engage tongue-shaped piece 202 in the engage holes 103a and 103b provided in the second body section 103 is cancelled, the sliding section 201 assumes a state of being accommodated most inwardly in the first body section 102 by a force of the coil spring 203.

Note that in a case where the wrist-type blood pressure meter cuff fastener 2 is again mounted on a wrist, the first body section 102, the second body section 103 and the third body section 104 are caused to be into the folded state, thereby enabling the wrist-type blood pressure meter cuff fastener 1 to be mounted on the wrist with ease. The sliding section 201 is in the state of being accommodated most inwardly in the first body section 102, thereby enabling the measuring winding length state to be reproduced in an instant.

### [Action and Effect]

In a wrist-type blood pressure meter cuff fastener 2 in this embodiment as well, it is possible to obtain an action and effect similar to those in the wrist-type blood pressure meter cuff fastener 1 in the first embodiment.

In a case of this embodiment, since the sliding section 201 assumes the state of being accommodated most inwardly in the first body section 102 when the wrist-type blood pressure meter cuff fastener 2 is in the mount/demount length state, the wrist-type blood pressure meter cuff fastener 2 assumes the measuring winding length state when being mounted on a wrist; therefore, an unintentional non-transition from the non-measuring winding length state to the measuring winding length state can be avoided in measurement of a blood pressure.

### (Third Embodiment)

Then, description will be given of a wrist-type blood pressure meter cuff fastener 3 in the third embodiment with reference to Fig. 9 to Fig. 14. The measuring wrist-type blood pressure meter fastener 3 in this embodiment includes: a belt member 101; a winding length adjusting unit 100C for adjusting a winding length of the wrist-type blood pressure meter fastener 3 with the belt member 101 around a wrist and is different from a wrist-type blood pressure meter fastener 1 or 2 in the first or second embodiment is the structure of the winding length adjusting unit 100C. Therefore, detailed description herein will be given of the structure of the winding length adjusting unit 100C. The same reference marks are attached to the same constitutes as in the winding length adjusting unit 100A or 100B and none of descriptions thereof is duplicated.

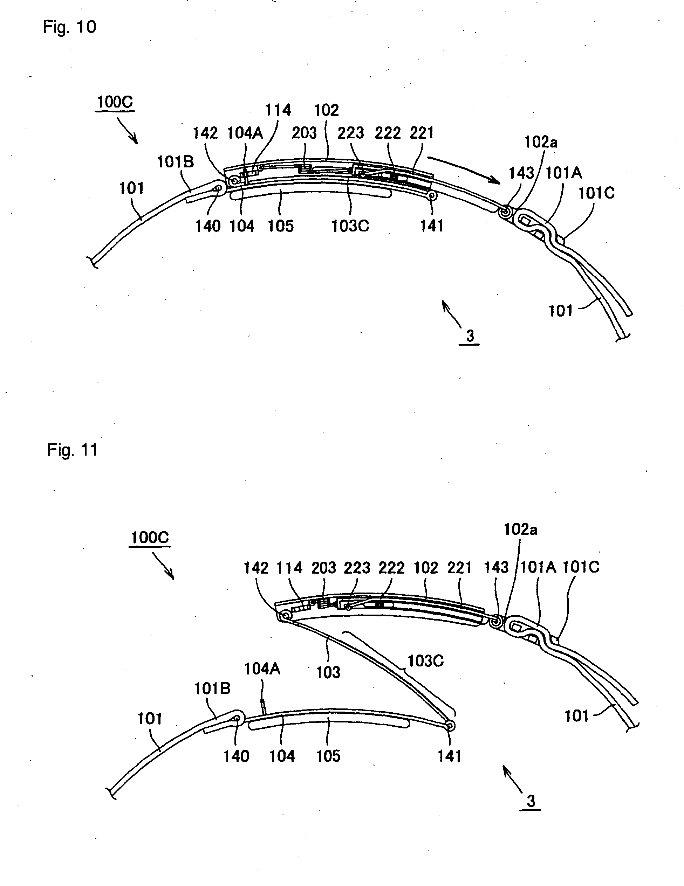

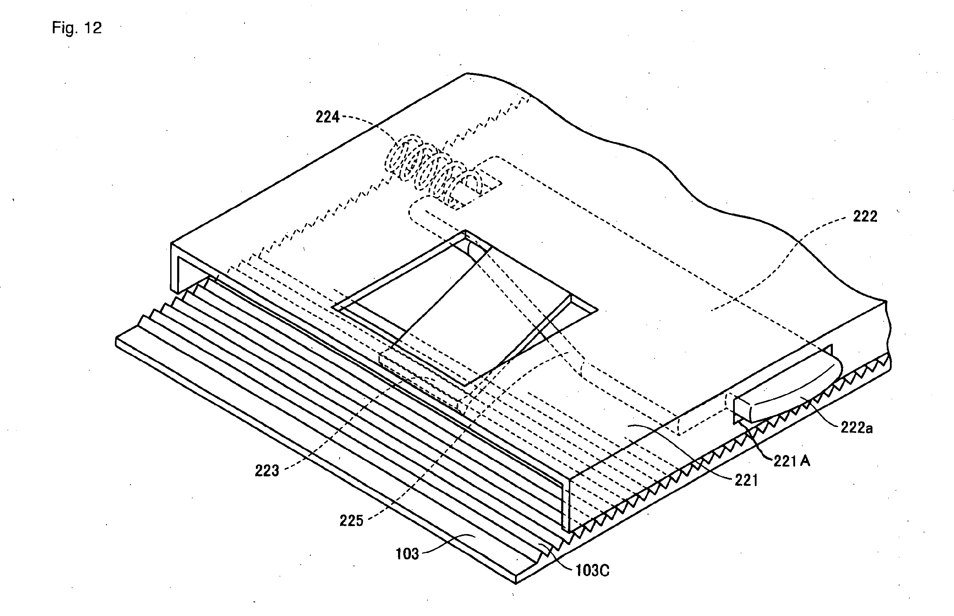

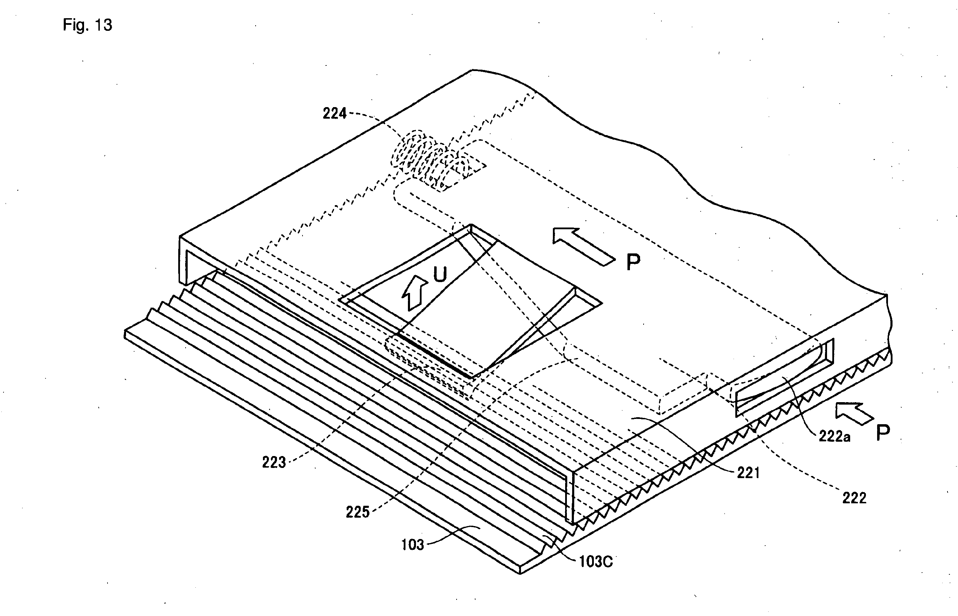

Note that Fig. 9 is a longitudinal sectional view of the winding length adjusting unit 100C showing a measuring winding length state of the wrist-type blood pressure meter cuff fastener 3 in this embodiment, Fig. 10 is a longitudinal sectional view of the winding length adjusting unit 100C showing a non-measuring winding length state of the wrist-type blood pressure meter cuff fastener 3 in this embodiment and Fig. 11 is a longitudinal sectional view of the winding length adjusting unit 100C showing a mount/demount length state of the wrist-type blood pressure meter cuff fastener 3 in this embodiment. Fig. 12 is a first partially enlarged view showing an internal structure of a first fixing mechanism in this embodiment and Fig. 13 is a second partially enlarged view showing the internal structure of the first fixing mechanism. Note that the body apparatus of the wrist-type blood pressure meter is omitted in the figures.

### [Winding Length Adjusting Unit 100C]

The winding length adjusting unit 100C in this embodiment, as shown in Fig. 11, has the same basic construction as the winding length adjusting unit 100B of the second embodiment. The winding length adjusting unit 100C is different from the winding length adjusting unit 100B in an internal structure of a first fixing mechanism. Herein, description will be given of the internal structure of a first fixing mechanism below with reference to Fig. 12 and Fig. 13.

### (Structure of First Fixing Mechanism)

A sliding section 221 provided slidably in the interior of a first body section 102 is provided with an engage tongue-shaped piece 223 extending obliquely downwardly. Provided on a surface of the second body section 103 is a region 103C with plural depressions and protrusions extending in parallel with a direction intersecting with the length direction of a second body section 103 and the engage tongue-shaped piece 223 elastically engages with the region 103C with depressions and protrusions.

Provided in the interior of the sliding section 221 is a lock cancel button 222 slidable in a direction in which the region 103C with depressions and protrusions extend and the head section 222a of the lock cancel button 222 is disposed so as to be exposed outwardly through an opening 221A formed in a sidewall of the sliding section 221. A coil spring 224 is disposed between the other side of the lock cancel button 222 from the head section 222a thereof and a sidewall of the sliding section 221, and the head section 222a of the lock cancel button 222 is pushed by the coil spring 224 in a direction of being protruded outwardly. A tapered sidewall 225 a width of which becomes gradually narrower toward the coil spring 224 side is provided to the lock cancel button 222 and disposed so as to be brought into contact with the lower surface side of the engage tongue-shaped piece 223.

In the first fixing mechanism with the above construction, the engage tongue-shaped piece 223, in a normal state, engages elastically with the depression and protrusion section 103C as shown in Fig. 12, thereby holding a locked state of the sliding section 221. On the other hand, as shown in Fig. 13, the head section 222a of the lock cancel button 222 is pushed inwardly (a direction P in the figure) against a force of the coil spring 224, thereby moving the tapered sidewall 225 in the direction P in the figure as well. As a result, the engage tongue-shaped piece 223 in contact with the tapered sidewall 225 is pushed upwardly (in a direction U in the figure) to release the engage tongue-shaped piece 223 from engagement with the depression protrusion region 103C, thereby releasing the sliding section 221 from the locked state.

Note that the detailed structure of the second fixing mechanism is similar to that of the first or second embodiment; therefore, none of descriptions thereof is duplicated.

### [Operation Procedure]

Description will be given of an operation procedure in a case where the wrist-type blood pressure meter cuff fastener 3 with the above structure is used with reference to Fig. 9 to Fig. 11.

### <Measuring Winding Length State>

First of all, the lock cancel buttons 114 and 114 of the second fixing mechanism are pushed inwardly to obtain the mount/demount length state, which is shown in Fig. 11, of the wrist-type blood pressure meter cuff fastener 3 to cause a wrist to get thereinto. In this step, the sliding section 221 assumes a state of being accommodated most inwardly in the first body section 102 by a force of the coil spring 203 since the engage tongue-shaped piece 223 is in no contact with the depression and protrusion region 103C.

The cuff 105 is positioned at a measuring position and not only is a length of the belt member 101 adjusted in the opening 102a, but the first body section 102, the second body section 103 and the third body section 104 are also caused to be in a folded state. In this context, the first body section 102, the second body section 103 and the third body section 104 are fixed into the folded state by the second fixing mechanism. In this step, the engage tongue-shaped piece 223 engages with the depression and protrusion region 103C provided on the second body section 103. Thereby, the blood pressure meter cuff fastener 3 assumes the measuring winding length state shown in Fig. 9 (the first winding length). Note that in this step, it is considered that a case arises where a winding length of the blood pressure meter cuff fastener 3 is improper. In this case, the lock cancel button 222 is pushed inwardly to release the engage tongue-shaped piece 223 from engagement with the depression and protrusion region 103C, thereby enabling fine adjustment movement of the sliding section 221 to be realized. Thereafter, a blood pressure is measured with the body apparatus of the wrist-type blood pressure meter.

### <Non-measuring Winding Length State>

Then, the lock cancel button 222 is operated in the first fixing mechanism to cancel a locked state of the sliding section 221 to slide the sliding section 221 in a direction in which the sliding section 221 is, as shown in Fig. 10, released from the first body section 102. Thereafter, the engage tongue-shaped piece 223 engages with the depression and protrusion region 103C provided on the second body section 103. By doing so, a winding length becomes longer than the first winding length to cause the blood pressure meter cuff fastener 3 to assume the non-measuring winding length state shown in Fig. 10 (the second winding length). As a result, a feeling of restraint in a wrist can be eliminated while the wrist-type blood pressure meter cuff fastener 3 is mounted on the wrist.

Note that in a case where a blood pressure is measured for the second time, the sliding section 221 is slid in a direction in which the sliding section 221 is accommodated into the first body section 102, thereby enabling the first winding length in the measuring winding length state set at the start of measurement to be reproduced with ease.

### <Mount/Demount Length State>

In a case where the wrist-type blood pressure meter cuff fastener 3 is taken way from a wrist, the lock cancel button 114 of the second fixing mechanism is operated to, as shown in Fig. 11, release the first body section 102, the second body section 103 and the third body section 104 from the folded state. With the release, the wrist-type blood pressure meter cuff fastener 3 can be easily taken away from a wrist. In this step, since engagement of the engage tongue-shaped piece 223 with the depression and protrusion region 103C provided on the second body section 103 is cancelled, the sliding section 221 assumes a state of being accommodated most inwardly in the first body section 102 by a force of the coil spring 203.

Note that in a case where the wrist-type blood pressure meter cuff fastener 3 is again mounted on a wrist, the first body section 102, the second body section 103 and the third body section 104 are caused to be in the folded state, thereby enabling the wrist-type blood pressure meter cuff fastener 3 to be mounted on the wrist with ease.

### [Action and Effect]

In a wrist-type blood pressure meter cuff fastener 3 in this embodiment as well, it is possible to obtain an action and effect similar to those in the wrist-type blood pressure meter cuff fastener 1 or 2 in the first or second embodiment.

In this embodiment, since in a case where the wrist-type blood pressure meter cuff fastener 3 is placed in the measuring winding length state, fine adjustment of an engaging position of the engage tongue-shaped piece 223 with the depression and protrusion region 103C can be conducted in continuous movement so as to be adapted for a wrist size while the sliding section 221 obtains a predetermined winding force, adjustment to a predetermined finding force can be easily realized even if a size of part of a human body changes slightly.

### (Fourth Embodiment)

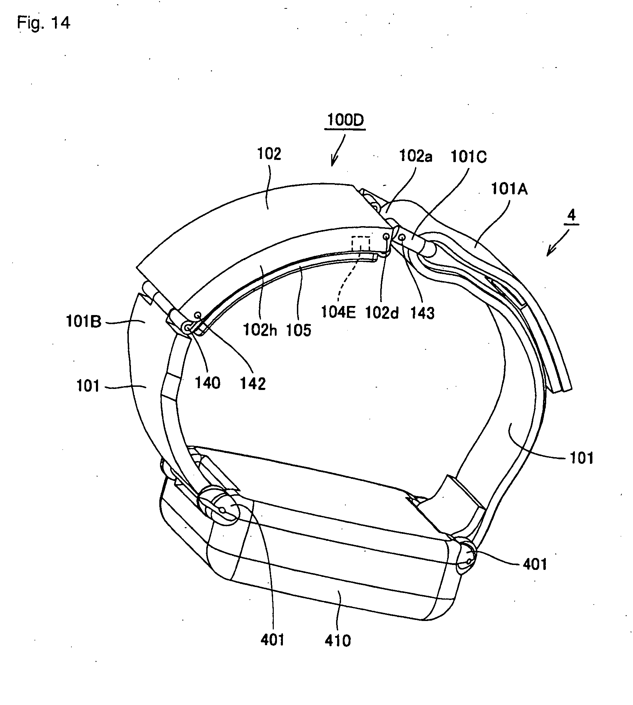

Then, description will be given of a wrist-type blood pressure meter cuff fastener 4 in the fourth embodiment with reference to Fig. 14 to Fig. 21. The wrist-type blood pressure meter fastener 4 in this embodiment includes: a belt member 101; a winding length adjusting unit 100D for adjusting a winding length of the wrist-type blood pressure meter fastener 4 with the belt member 101 around a wrist, and is different from each of the wrist-type blood pressure meter fasteners 1 to 3 in the first to third embodiments is in the structure of the winding length adjusting unit 100D. Therefore, detailed description will be given of the structure of the winding length adjusting unit 100D. The same reference marks are attached to the same constitutes as in each of the winding length adjusting unit 100A to 100C and none of descriptions thereof is duplicated. Note that the body apparatus 410 of the wrist- type blood pressure meter is attached to the belt member 101 with a pivot section 401 interposed therebetween.

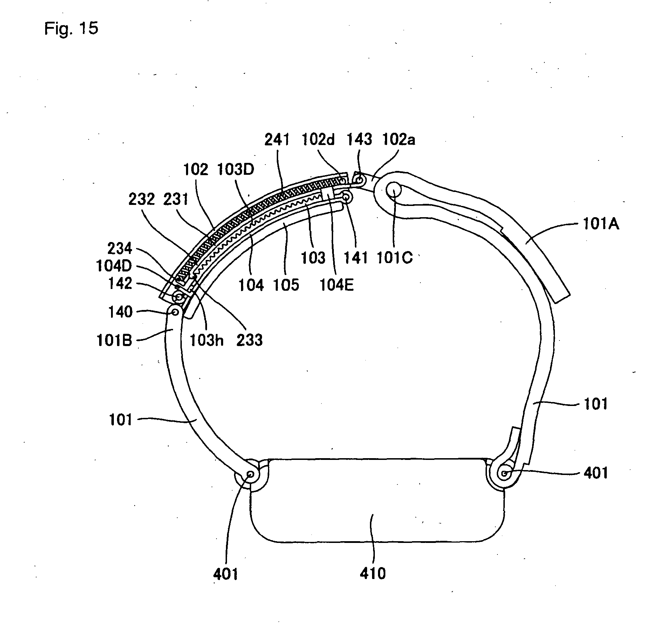

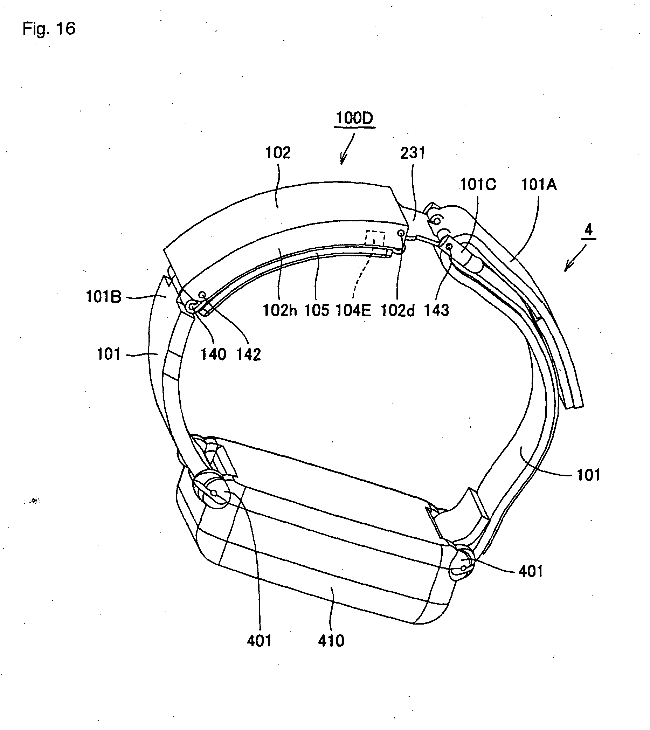

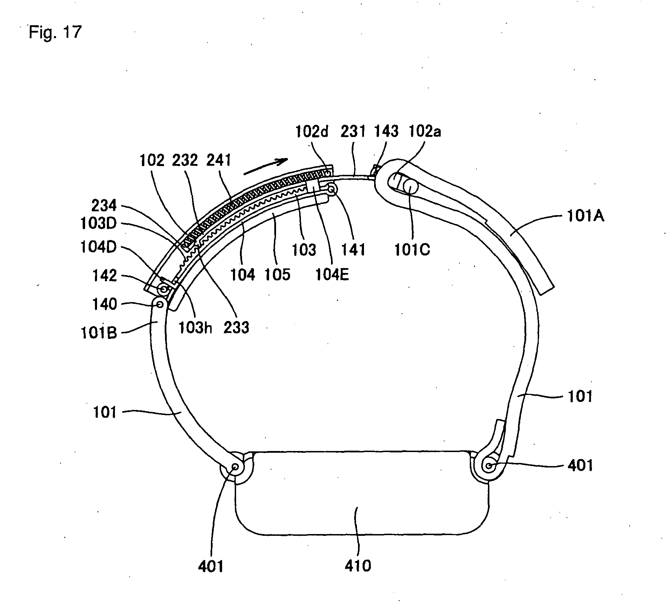

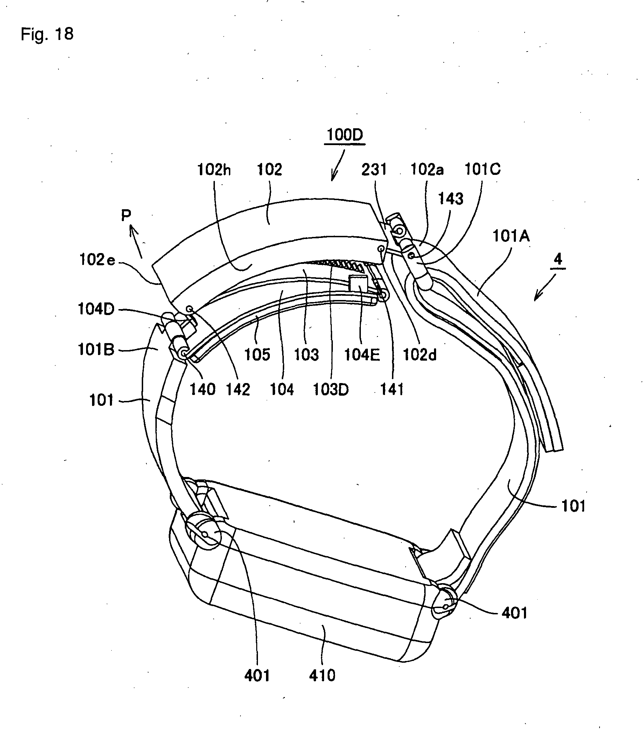

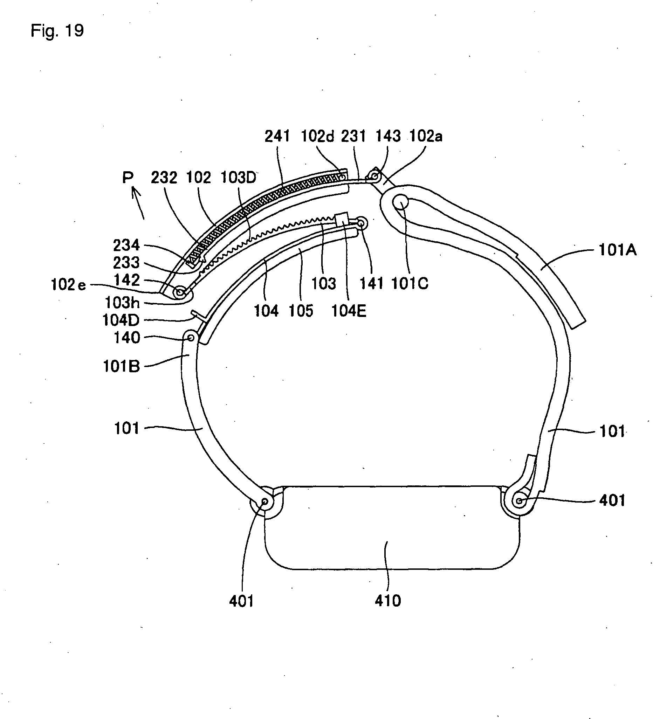

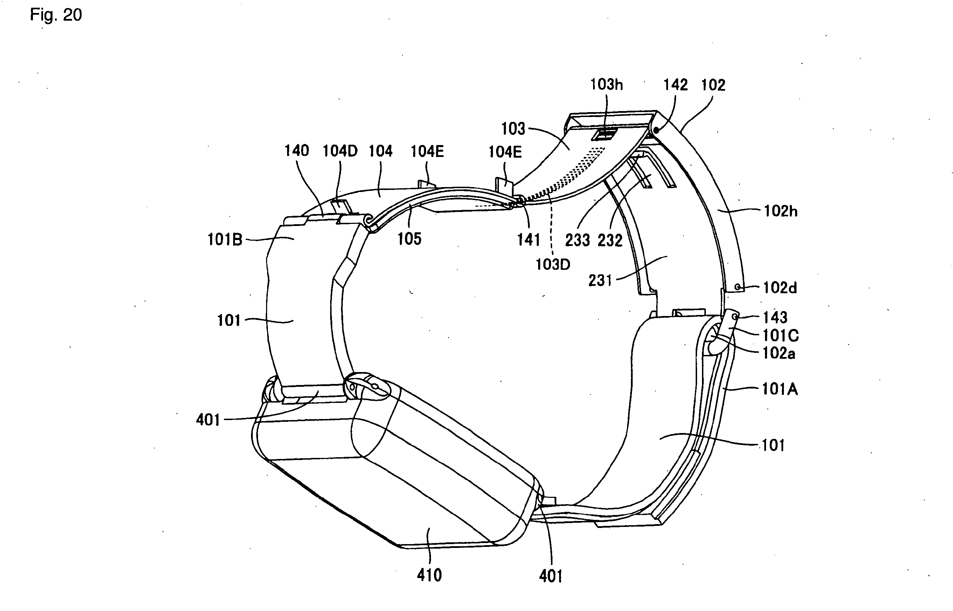

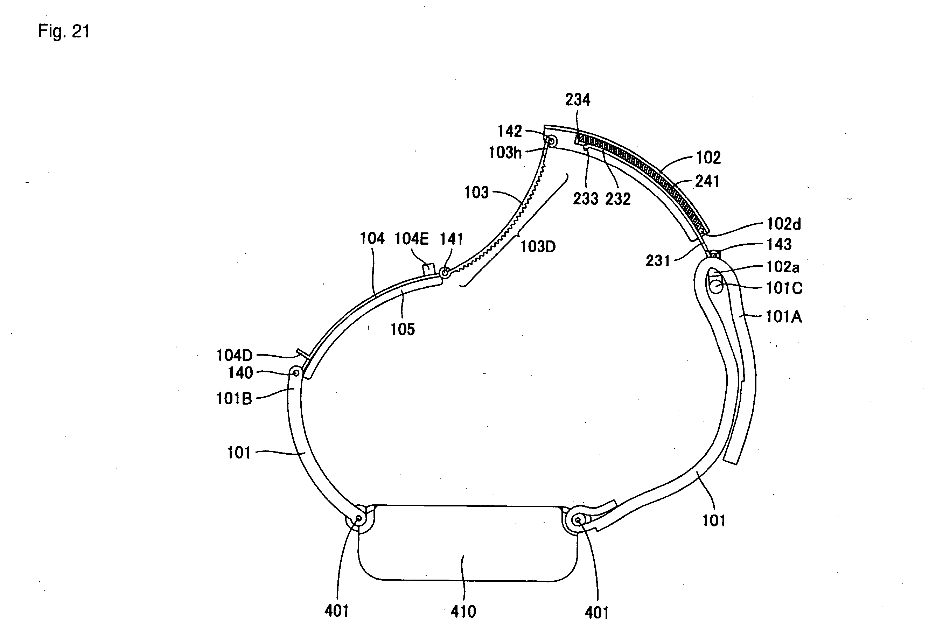

Fig. 14 is a perspective view showing a measuring winding length state of all of the wrist-type blood pressure cuff fastener 4 in this embodiment, Fig. 15 is a longitudinal sectional view showing a measuring winding length state of the wrist-type blood pressure cuff fastener 4 in this embodiment, Fig. 16 is a perspective view showing a non-measuring winding length state of all of the wrist-type blood pressure cuff fastener 4 in this embodiment, Fig. 17 is a longitudinal sectional view showing the non-measuring winding length state of the wrist-type blood pressure cuff fastener 4 in this embodiment, Fig. 18 is a perspective view showing adjustment of a non-measuring winding length of all of the wrist-type blood pressure cuff fastener 4 in this embodiment, Fig. 19 is a longitudinal sectional view showing adjustment of a non-measuring winding length of the wrist-type blood pressure cuff fastener 4 in this embodiment, Fig. 20 is a perspective view showing a mount/demount length state of all of the wrist-type blood pressure cuff fastener 4 in this embodiment and Fig. 21 is a longitudinal sectional view showing the mount/demount length state of the wrist-type blood pressure cuff fastener 4 in this embodiment.

### [Winding Length Adjusting Unit 100D]

The winding length adjusting unit 100D in this embodiment has the same basic construction as the winding length adjusting unit 100C of the third embodiment. The winding length adjusting unit 100D is different from the winding length adjusting unit 100C in the internal structures of first and second fixing mechanisms.

### (Structure of First Fixing Mechanism)

A sliding section 231 provided slidably in the interior of a first body section 102 is provided with an engage tongue-shaped piece 232 as shown in Fig. 18 and Fig. 19. An engage protrusion 233 is provided at the distal end of the engage tongue-shaped piece 232. Provided on a surface of the second body section 103 is a region 103D with plural depressions and protrusions extending in parallel with a direction intersecting with the length direction of the second body section 103 and the engage protrusion 233 is provided so as elastically engage with the region 103D with depressions and protrusions. On the other hand, a connecting member 101C having an opening 102a is connected pivotably around a pivot section 143 to the other end side of the sliding section 231. By causing one end side 101A of the belt member 101 to pass through the opening 102a, not only is the one end side 101A of the belt member 101 fixed there, but adjustment of a length of the belt member 101 is also enabled.

The construction is such that a compression coil spring 241 is provided in a space between the first body section 102 and the sliding section 231 and one end of the compression coil spring 241 is brought into contact with a sidewall surface 234 provided on the second body section 103 side at the one side end of the sliding section 231, while the other end of the compression coil spring 241 is brought into contact with a pin 102d provided in the other side of the first body section 102 from a pivot section 142.

In the first fixing mechanism with the above construction, the engage tongue-shaped piece 233, in a normal state, engages elastically with the depression and protrusion section 103D as shown in Fig. 15, thereby holding a locked state of the sliding section 231. On the other hand, as shown in Fig. 20 and Fig. 21, the first body section 102, the second body section 103 and the third body section 104 are released from a folded state and thereby, the engage protrusion 233 is released from the engagement with the depression and protrusion region 103D and in turn, the sliding section 231 is released from the locked state. In this state, the sliding section 231 is slidable, from a position at which the sidewall surface 234 is brought into contact with the pivot section 142 of the first body section 102, that is a position at which the sliding section 231 is accommodated most inwardly in the first body section 102, up to a position at which the sliding member 231 is released from the first body section 102 till the compression coil spring 241 reaches the most compressed state.

### (Structure of Second Fixing Mechanism)

Then, description will be given of the structure of the second fixing mechanism.

The engage tongue-shaped piece 104D is, as shown in Fig. 18 and Fig. 19, provided to the third body section 104 in the vicinity of the pivot section 140. The engage tongue-shaped piece 104D passes through an opening 103h formed in the second body section 103 to engage with side faces of the pivot section 142 of the first body section 102 in a folded state of the first body section 102, the second body section 103 and the third body section 104 in a similar manner to that of the lock pin 104A in each of the first to third embodiments.

Provided at both side surfaces of the pivot section 141 side of the third body section 104 are a pair of engaging erected walls 104E not only elastically hugging the second body section 103 at the side surface thereof with the inner surface sides of the engage erected walls 104E, but being brought into elastic contact with the inner surfaces 102h of the first body section 102 with the outer surface sides of the engage erected walls 104E in the folded state of the first body section 102, the second body section 103 and the third body section 104.

In the second fixing mechanism with the above construction, the engagement of the engage tongue-shaped piece 104D and the pivot section 142 of the first body section 102, contact of the engaging elected walls 104E with the side surfaces of the second body section 103, and contact of the engaging elected walls 104E with the side walls 102h of the first body section 102 sustain the folded state of the first body section 102, the second body section 103 and the third body section 104 (the measuring winding length state and the non-measuring winding length state shown in Fig. 14 to Fig. 17).

On the other hand, in a case where the end section 102e in the pivot section 142 side of the first body section 102 is pulled out outwardly (in a direction of an arrow mark P shown in the figures), cancellation is effected of engagement of the engage tongue-shaped 104D with the pivot section 142 of the first body section 102, contact of the engage elected walls 104E with the side surfaces of the second body section 103, and contact of the engaging elected walls 104E with the side walls 102h of the first body section 102, and the first body section 102, the second body section 103 and the third body section 104 are released from the folded state (themount/demount length state shown in Fig. 20 and Fig. 21).

### [Operation Procedure]

Description will be given of an operation procedure in a case of employing the wrist-type blood pressure meter cuff fastener 4 with the above structure with reference to Fig. 14 to Fig. 21.

### <Measuring Winding Length State>

First of all, the end section 102e in the pivot section 142 side of the first body section 102 is pulled outwardly to cause the wrist-type blood pressure meter cuff fastener 4 to assume the mount/demount length state shown in Fig. 20 and Fig. 21 and to cause a wrist to get into the wrist-type blood pressure meter cuff fastener 4. In this step, since the engage protrusion 233 is in no contact with the depression and protrusion region 103D, the sliding section 231 assumes a state of being accommodated most inwardly in the first body section 102 by a force of the compression coil spring 241.

The cuff 105 is positioned at a measuring position and not only is a length of the belt member 101 adjusted in the opening 102a, but the first body section 102, the second body section 103 and the third body section 104 are also caused into a folded state. In this context, the first body section 102, the second body section 103 and the third body section 104 are fixed into the folded state by the second fixing mechanism. In this step, the engage protrusion 233 engages with the depression and protrusion region 103D provided on the second body section 103. Thereby, the wrist-type blood pressure meter cuff fastener 4 assumes the measuring winding length state shown in Fig. 14 and Fig. 15 (the first winding length). In this step, an engaging position of the engage protrusion 233 with the depression and protrusion region 103D provided on the second body section 103 is determined by a result of adjustment of a length of the belt member 101 and a force of the compression coil spring 241. Thereafter, a blood pressure is measured with the body apparatus 410 of the wrist-type blood pressure meter.

### <Non-measuring Winding Length State>

Then, the end section 102e in the pivot section 142 of the first body section 102 is pulled outwardly to temporarily cancel engagement of the engage tongue-shaped piece 104D of the second fixing mechanism with the pivot section 142 of the first body section 102 (the state shown in Fig. 18 and Fig. 19). Then, the first body section 102, the second body section 103 and the third body section 104 is released from the folded state and in turn, engagement of the engage protrusion 233 of the first fixing mechanism with the depression and protrusion region 103D provided on the second body section 103 is cancelled. Note that while in this state, engagement of the engaging erected wall 104E with the side surfaces 102h is cancelled, elastic engagement of the engaging erected walls 104E with the side surfaces of the second body section 103 is sustained; therefore, there is no chance that the second body section 103 and the third body section 104 assume a largely open state.

In this state, the sliding section 231 is slid in a direction of being released from the first body section 102 by a proper distance to cause the first body section 102, the second body section 103 and the third body section 104 to assume a folded state there. In this context, the first body section 102, the second body section 103 and the third body section 104 are fixed in the folded state by the second fixing mechanism. While, in this step, the engage protrusion 233 engages with the depression and protrusion region 103D provided on the second body section 103, the engagement is effected at a position to which the sliding section 231 is slid as compared with the measuring winding length state. With such engagement, a winding length is longer than the first winding length and the wrist-type blood pressure meter cuff fastener 4 assumes the non-measuring winding length state shown in Fig. 16 and Fig. 17 (the second winding length). As a result, a user can be released from a feeling of restraint around a wrist while the wrist-type blood pressure meter cuff fastener 4 is mounted on the wrist.

Note that in a case where a blood pressure is measured for the second time, the end section 102e in the pivot section 142 side of the first body section 102 is again pulled outwardly to cancel engagement of the engage tongue-shaped piece 104D of the second fixing mechanism with the pivot section 142 of the first body section 102 and to thereby cause the first body section 102, the second body section 103 and the third body section 104 to assume a folded state without sliding the sliding section 231, thereby enabling the first winding length in the measuring winding length state set at the start of measurement to be reproduced with ease.

### <Mount/Demount Length State>

In a case where the wrist-type blood pressure meter cuff fastener 4 is taken way from a wrist, the end section 102e in the pivot section 142 side of the first body section 102 is pulled outwardly to thereby cause engagement of the engage tongue-shaped piece 104D with the pivot section 142 of the first body section 102, engagement of the engaging erected wall 140E with the side surfaces of the second body section 103 and engagement of the engaging erected wall 104E with the side walls 102h of the first body section 102 to be cancelled and in turn, release the first body section 102, the second body section 103 and the third body section 104 from the folded state as shown in Fig. 20 and Fig. 21. With the release, the wrist-type blood pressure meter cuff fastener 4 can be easily taken away from a wrist. In this step, since engagement of the engage protrusion 233 with the depression and protrusion region 103C provided on the secondbody section 103 is cancelled, the sliding section 231 assumes a state of being accommodated most inwardly in the first body section 102 by a force of the coil spring 241.

Note that in a case where the wrist-type blood pressure meter cuff fastener 4 is again mounted on a wrist, the first body section 102, the second body section 103 and the third body section 104 are caused to be into the folded state, thereby enabling the wrist-type blood pressure meter cuff fastener 4 to be mounted on the wrist with ease.

### [Action and Effect]

In a wrist-type blood pressure meter cuff fastener 4 in this embodiment as well, an action and effect similar to those in the wrist-type blood pressure meter cuff fastener 1, 2 or 3 in the first, second or third embodiment.

In this embodiment, with a mechanism switching between engagement and cancellation of a lock pin of the first fixing mechanism or the second fixing mechanism provided, it is possible to make the construction of the wrist-type blood pressure meter fastener 4 to be extremely simple.

The embodiments disclosed herein are presented by way of illustration in every respect, but do not constitute any basis for restricted interpretation. Accordingly, the technical scope of the invention should not be interpreted by the embodiments described above in a limited way but defined based on the description in the appended claims. The technical scope of the invention includes all of alterations or modifications of the embodiments in a meaning and scope equivalent to the scope of the claims.

According to a blood pressure meter cuff fastener based on the invention, it is easily to select one of the three states including the measuring winding length state, the non-measuring winding length state and the mount/demount length state with ease; therefore, a burden imposed on a human body in a normally mounted state of the blood pressure meter cuff fastener is alleviated, thereby enabling release froma feeling of restraint of being clamped for a long time to be realized. In measurement of a blood pressure as well, a winding length can be reproduced with correctness, thereby enabling reliability in blood pressure measurement to be improved.

## Claims

1. A blood pressure meter cuff fastener used for fastening a blood pressure meter cuff on a human body comprising:
a belt member wound around a part of a human body and
winding length adjusting unit, connected to the belt member, and for adjusting a winding length of the blood pressure meter cuff fastener with the belt member thereof around the part of a human body, wherein
the winding length adjusting unit is provided so as to be capable of selecting one of three states including
a measuring winding length state adjusting the blood pressure meter cuff fastener to a first winding length for measuring a blood pressure in the part of a human body,
a non-measuring winding length state adjusting the blood pressure meter cuff fastener to a second winding length longer than the first winding length in order to maintain a mounting state thereof on the part of a human body in a non-measuring state thereof for a blood pressure and
a mount/demount length state capable of mounting or demounting the blood pressure meter cuff fastener on the part of a human body.

2. The blood pressure meter cuff fastener according to claim 1, wherein the winding length adjusting unit has the body section and a sliding section slidably provided to the body section, wherein
the sliding section is slid in a direction in which the sliding section is accommodated into the body section to thereby enable the measuring winding length state to be acquired,
while being slid in a direction in which the sliding section is released from the body section to thereby enable the non-measuring winding length state to be acquired.

3. The blood pressure meter cuff fastener according to claim 2, wherein the winding length adjusting unit has a first fixing mechanism for selectively fixing one of the measuring winding length state and the non-measuring winding length state between the body section and the sliding section.

4. The blood pressure meter cuff fastener according to claim 2 or 3, wherein the body section has
a first body section,
a second body section provided pivotably on the other end side of the first body section so as to be folded on the first body section, and
a third body section provided pivotably on the other side of the second body section from the side on which the first body section thereof is provided so as to be folded on the second body section, wherein
the first body section, the second body section and the third body section are folded so as to be superimposed one on another to thereby enable the measuring winding length state and the non-measuring winding length state to be acquired,
while being released from the folding state of the first body section, the second body section and the third body section to thereby enable the mount/demount length state to be acquired.

5. The blood pressure meter cuff fastener according to claim 4, wherein a second fixing mechanism for fixing the measuring winding length state and the non-measuring winding length state is provided between the first body section and the third body section.

6. An electronic blood pressure meter having a blood pressure meter cuff fastener according to any of claims 1 to 5.
